# FASCICULE DE BREVET EUROPEEN

(11) **EP 1 390 513 B1**
(45) Date de publication et mention de la délivrance du brevet: **28.05.2008**
(21) Numéro de dépôt: 02740794.9
(22) Date de dépôt: 17.05.2002
(51) Int. Cl.: C12N 15/65, C12N 15/64

(54) **PROCEDE DE CLONAGE PAR DOUBLE SELECTION ET VECTEURS POUR CE PROCEDE**
VERFAHREN ZUR KLONIERUNG MITTELS DOPPELTER SELEKTION UND VEKTOREN DAFÜR
DOUBLE SELECTION CLONING METHOD AND VECTORS THEREFOR

(30) Priorité: 18.05.2001 FR 0106568
(43) Date de publication de la demande: 25.02.2004
(73) Titulaire: Genoway, 69007 Lyon (FR)
(72) Inventeur: BERTAUX, Fabien, F-69290 Grezieu La Varenne (FR); LAGARDETTE, Florence, F-69460 Saint Etienne Les Oullie (FR); LAPIZE, Christine, F-38200 Vienne (FR)
(74) Mandataire: Warcoin, Jacques
(86) Numéro de dépôt international: PCT/FR2002/001682
(87) Numéro de publication internationale: WO 2002/095037

(56) Documents cités:
- EP-A- 0 426 455
- EP-A- 0 814 165
- WO-A-01/04306
- WO-A-01/31039
- WO-A-90/11354
- WO-A-99/23239
- LE MOUELLIC H ET AL: "TARGETED REPLACEMENT OF THE HOMEOBOX GENE HOX-3.1 BY THE ESCHERICHIA-COLI LACZ IN MOUSE CHIMERIC EMBRYOS" PROCEEDINGS OF THE NATIONAL ACADEMY OF SCIENCES OF THE UNITED STATES, vol. 87, no. 12, 1990, pages 4712-4716, XP002189922 1990 ISSN: 0027-8424 cité dans la demande
- YAVUZER U ET AL: "PWITCH: A VERSATILE TWO-HYBRID ASSAY VECTOR FOR THE PRODUCTION OF EPITOPE/ACTIVATION DOMAIN-TAGGED PROTEINS BOTH IN VITRO AND IN YEAST" GENE, ELSEVIER BIOMEDICAL PRESS. AMSTERDAM, NL, vol. 165, 1995, pages 93-96, XP000605469 ISSN: 0378-1119
- GABANT P ET AL: "New positive selection system based on the parD (kis/kid) system of the R1 plasmid" BIOTECHNIQUES, EATON PUBLISHING, NATICK, US, vol. 28, no. 4, avril 2000 (2000-04), pages 784-788, XP000910383 ISSN: 0736-6205

## Description

La présente invention se rapporte à un nouveau procédé de clonage d'un fragment d'ADN dans un vecteur, par une sélection visuelle en utilisant deux antibiotiques, ainsi qu'aux vecteurs utilisables pour la mise en oeuvre de ce procédé, et à un kit comprenant ces vecteurs.

Le clonage d'ADN dans un vecteur consiste à insérer un ou plusieurs fragments d'ADN successivement ou non dans un vecteur afin de construire un nouveau vecteur. Lors de ces étapes de construction, on peut être confronté à de nombreux problèmes, parmi lesquels on peut citer un faible taux d'efficacité des réactions de ligation lors de l'introduction du fragment d'ADN, une instabilité du vecteur ayant intégré le fragment d'ADN induisant une sur-représentation du vecteur de départ...

Ces difficultés peuvent être observées pour tout type de vecteurs, tels les plasmides, les cosmides, les chromosomes artificiels (de bactéries, BAC, de levure, YAC, ou mammifères MAC), et en particulier pour des constructions complexes telles que la construction de vecteurs de recombinaison homologue, pour lesquels on recherche l'introduction de fragments de grande taille (généralement entre 2 et 30 kilobases (kb), de préférence entre 2 et 7 kb) dans des vecteurs appropriés. Il peut être important d'intégrer, dans un vecteur pour la recombinaison homologue, deux fragments d'ADN différents consistant en les régions 3' et 5' du locus que l'on vise par recombinaison.

*La demande* WO 99/23239 *décrit une méthode de construction de vecteurs par recombinaison homologue, qui nécessite la présence de séquences homologues sur le vecteur et la cassette à insérer*.

Il est notamment également préférable que ces vecteurs puissent être sélectionnés à la fois dans l'hôte de clonage (souvent un hôte inférieur, procaryote ou eucaryote unicellulaire, l'hôte le plus fréquent étant *Escherichia coli)* et dans la cellule mammifère cible (par exemple une cellule ES, de préférence murine ou issue d'un rongeur). En général, on recherche donc que les vecteurs présentent des gènes de sélection (notamment de résistance aux antibiotiques) fonctionnels dans les deux organismes.

Il existe aujourd'hui quelques techniques permettant de sélectionner les événements d'insertion d'un fragment d'ADN dans un vecteur, notamment la sélection blanc/bleu, le fragment d'ADN étant inséré dans le gène lacZ, et menant à l'extinction de l'activité dudit gène (Ullman et al, J. Mol. Biol. 1967, 24, 339-43). Toutefois, dans les cas d'inserts difficiles à cloner, et même si l'on peut observer aisément les événements positifs de manière visuelle, le bruit de fond reste important.

On peut aussi utiliser la suppression de l'activité du ccdB (Bernard et al, Gene, 1994, 148, 71-4). Toutefois, la préparation du plasmide avant le clonage nécessite d'utiliser une autre souche d'*E*. *coli* (gyrA462) que la souche dans laquelle on effectuera la sélection après clonage.

Les deux méthodes décrites ci-dessus ne permettent pas non plus de savoir si le fragment d'ADN inséré l'a été dans l'orientation souhaitée.

La présente invention se rapporte à un procédé de clonage (insertion) d'un fragment d'ADN dans un vecteur, ledit procédé permettant d'éliminer une part importante des événements ne résultant pas de la réaction d'insertion désirée. Il est à noter que le procédé selon l'invention permet également de sélectionner les événements de clonage de l'ADN dans l'orientation désirée.

Ainsi, la présente invention a pour objet un procédé de clonage d'un fragment d'ADN dans un vecteur A, ledit vecteur A comprenant un gène de résistance à un antibiotique I fonctionnel dans les cellules I hôtes de clonage, et un promoteur P fonctionnel dans les cellules I hôtes de clonage, comprenant les étapes consistant à :
a) intégrer de l'ADN dans le site polylinker d'un vecteur B utilisable dans des cellules II hôtes de clonage, ledit site polylinker étant localisé dans une cassette située entre deux sites de restrictions identiques ou différents, ladite cassette comprenant un gène III conférant une résistance à un antibiotique III dans les cellules I hôtes de clonage, ledit gène III n'étant pas sous le contrôle d'un promoteur lui permettant d'être actif dans les cellules I hôtes de clonage, ledit vecteur B présentant un gène II, actif dans les cellules II hôtes de clonage, de résistance à un antibiotique II,
b) exciser ladite cassette du vecteur B par coupure avec les enzymes de restriction correspondant auxdits sites de restriction,
c) effectuer une ligation de ladite cassette excisé dans ledit vecteur A linéarisé de telle sorte que ladite cassette est insérée sous le contrôle dudit promoteur I fonctionnel dans les cellules I hôtes de clonage,
d) sélectionner les événements de ligation chez les cellules I hôtes de clonage résistantes à la fois à l'antibiotique I et à l'antibiotique III.

De préférence, les vecteurs A et B sont des plasmides, mais ces vecteurs peuvent également être des cosmides, ou des chromosomes artificiels.

Le site « polylinker » est un site permettant l'intégration de l'ADN extérieur, et comportant généralement quelques (entre 1-2 et 5-7) sites de restriction.

Il est intéressant de noter que la sélection étant effectuée par la double résistance aux antibiotiques I et III, ceci permet d'obtenir également un clonage orienté, dans la mesure où le gène III, pour être fonctionnel, doit être orienté de telle façon à se retrouver sous le contrôle du promoteur P.

Dans un mode de mise en oeuvre particulier de l'invention, les cellules I hôtes de clonage et les cellules II hôtes de clonage sont des cellules procaryotes, de préférence *Escherichia coli.* Toutefois, ces cellules peuvent être des cellules eucaryotes, les cellules I et II n'étant pas nécessairement identiques. Il est donc possible que les cellules II soient des levures et que les cellules I soient des bactéries. D'autres hôtes de clonages peuvent aussi être utilisés, par exemple des phages.

Dans un mode de réalisation préférée de l'invention, ledit gène III confère également une résistance à un antibiotique dans des cellules eucaryotes, notamment des cellules de mammifères, de préférence les cellules ES de rongeurs, souris, porcines, ovines, bovines, de lapin, ou humaines. Un gène particulièrement préféré est le gène de résistance à la kanamycine dans les cellules procaryotes, qui induit également une résistance à la néomycine dans les cellules eucaryotes. Il est intéressant que le gène III puisse être utilisé dans les deux types cellulaires, notamment pour sélectionner les événements de recombinaison homologues dans les cellules eucaryotes. L'homme du métier connaît différents gènes possédant ces propriétés, et on peut aussi citer, de façon on exhaustive, la zéocine, ou l'hygromycine B.

Il est à noter que dans un cas particulier de mise en oeuvre de l'invention, on utilise, au lieu d'un gène III de résistance à un antibiotique, un gène marqueur, qui permet aussi de vérifier la présence de l'insert. On peut notamment utiliser le gène lacZ ou le gène codant pour la GFP.

Dans un mode de réalisation particulier de l'invention, lesdits gènes I et II sont identiques. On peut choisir différents gènes de résistance à des antibiotiques, et notamment le gène induisant la résistance à l'ampicilline. Les gènes de résistance aux antibiotiques utilisables dans les vecteurs de clonage sont bien connus de l'homme du métier, et on peut notamment citer les gènes de résistance à l'hygromycine B, au chloramphénicol, à la tétracycline, à la zéocyne, sans que cette liste ne soit exhaustive.

Dans un mode de réalisation particulier de l'invention, lesdits sites de restriction flanquant la cassette contenant le polylinker dans le vecteur B sont rares.

Par « site de restriction rare », on entend un site de restriction dont la fréquence de coupure est supérieure à 10 kb, de préférence 15 kb, de façon plus préférée 20 kb dans le génome humain, murin, de l'organisme cible en général. Parmi les enzymes rares, on peut notamment citer *Pme*I, *SgrA*I, *Rsr*II, *Cla*I, *Not*I, *Asc*I, *Pac*I, *Srf*I, *Nhe*I, *Fse*I, *Nsi*I, *Sce*I. On peut aussi citer les sites des « Homing endonucleases ». Ces enzymes sont des protéines codées par des gènes possédant des introns s'auto-épissant. Ces enzymes effectuent des coupures site-spécifiques dans de l'ADN double brin, et reconnaissent généralement des sites de 18-20 bases ou plus. On note en particulier *I-Ppoi, I-Cre*I, *I-Ceu*I, *PI-Ps*I, *I-Sce*I, *PI-Sce*I. Ces enzymes sont dites « très rares ».

Dans un mode de réalisation particulier de l'invention, ledit vecteur A possède également un site polylinker permettant ou ayant permis l'insertion d'un fragment d'ADN. Ceci permet en effet de pouvoir effectuer le clonage des régions 5' et 3' du locus cible visé pour la recombinaison homologue plus facilement, puisqu'on intègre de façon préférentielle la région 3' ou la région 5' dans le plasmide A, avant d'utiliser le procédé selon l'invention avec l'autre région et le plasmide B. Il est alors intéressant que la linéarisation de A soit effectuée en utilisant une enzyme de restriction rare ou très rare, telles que définies plus haut.

L'homme du métier sait définir les promoteurs P actifs dans les hôtes de clonage I. Dans les modes de mise en oeuvre préférés de l'invention, ledit promoteur P est choisi parmi le promoteur pour le gène de résistance à la kanamycine du transposon Tn5, le promoteur du gène bla (résistance à l'ampicilline), le promoteur de l'opéron tryptophane Trp, le promoteur de l'opéron lactose, ou tout autre promoteur accessible dans la base de données PromEC (http://bioinfo.md.huji.ac.il/marg/promec).

Dans un cas particulier de mise en oeuvre de l'invention, les vecteurs selon l'invention possèdent également les caractéristique suivantes :
- ledit gène III dans ladite cassette dans le vecteur B est sous la dépendance d'un promoteur Eb actif dans les cellules eucaryotes, notamment mammifères, ou
- ledit vecteur A présente un promoteur Ea actif dans les cellules eucaryotes, notamment mammifères, situé de telle sorte que ledit gène III est sous le contrôle dudit promoteur Ea après insertion dans le vecteur A.

L'homme du métier connaît les promoteurs pouvant être utilisés dans les hôtes II de clonage. Dans les modes de réalisation préférés pour lesquels les hôtes II sont des cellules eucaryotes, lesdits promoteurs Ea et Eb sont choisis parmi les promoteurs de la bêta-actine de poulet, du PGK, de la thymidine kinase du virus d'herpes simplex, du SV40, ou bien le « immediate early enhancer » du cytomégalovirus humain.

Ce mode de réalisation est notamment préféré lorsque le gène III peut induire une résistance à un antibiotique à la fois dans les cellules procaryotes et les cellules eucaryotes, notamment de mammifères. Les promoteurs hybrides couplés procaryotes-eucaryotes sous le contrôle desquels se trouve alors ledit gène III après la mise en oeuvre du procédé sont similaires aux promoteurs décrits notamment pour les plasmides pEGFP-C1 dans Cormack et al (1996, Gene, 173, 33-8) ou pGN dans LeMouellic et al (1990, Proc. Natl. Acad. Sci. USA, 87,4712-6).

Ainsi qu'il a été précisé, il est souvent intéressant d'utiliser le procédé selon l'invention pour la préparation de vecteurs destinés à la recombinaison homologue dans des cellules souches d'organismes pluricellulaires. Ainsi on insère un premier fragment (par exemple 3' ou 5' d'un locus cible) dans le vecteur A, l'autre fragment dans le vecteur B, et on effectue la construction du vecteur final, de grande taille, par le procédé selon l'invention.

Ainsi, dans un cas particulier, les fragments d'ADN introduits dans ledit vecteur B et de façon optionnelle dans ledit vecteur A sont des fragments d'ADN génomique, de façon préférée issus d'un même hôte. Dans un cas particulièrement préféré, lesdits fragments sont les fragments 3' et 5' d'un même locus, qui est cible pour la recombinaison homologue.

L'hôte en question est de préférence un mammifère, mais peut aussi être une levure, un fongus ou une bactérie. Dans le cas des mammifères, on cite les rongeurs (notamment souris, rats, lapins), mais aussi les porcins, les ovins, les bovins, les canins, les félins.

Le procédé selon l'invention permet donc, selon les modes de mise en oeuvre :
- une sélection visuelle positive des clones ayant incorporé l'insert désiré, puisque seuls ceux-ci peuvent survivre sur le milieu de sélection,
- un gain de temps sur la construction du vecteur final puisque l'étape impliquant la création d'un vecteur de grande taille est celle où la sélection positive s'opère. De plus, l'insertion de deux inserts (par exemple 5' et 3' d'un même locus) s'effectue dans deux vecteurs différents lors de la première étape, non pas successivement.
- l'utilisation du gène de résistance III permet, quand il est fonctionnel à la fois dans les cellules procaryotes et eucaryotes, de pouvoir effectuer la réaction de recombinaison homologue dans les cellules eucaryotes directement après le clonage (éventuellement après linéarisation du vecteur final ou excision de l'insert), et de pouvoir aisément sélectionner les événements de recombinaison dans ces cellules.

Les vecteurs selon la présente invention sont également objets de la présente invention, seuls, ou en combinaison dans un kit, lui-même également objet de l'invention.

L'invention a donc aussi pour objet un kit pour le clonage de fragments d'ADN dans un vecteur A, comprenant :
- un vecteur A, basé sur un squelette de vecteur usuel de clonage dans des cellules I hôtes de clonage, ledit vecteur comprenant un gène de résistance à un antibiotique I fonctionnel dans lesdites cellules I, présentant de 5' en 3', :
   - éventuellement un polylinker permettant l'introduction de fragments d'ADN dans ledit vecteur,
   - un promoteur P fonctionnel dans les cellules I hôtes de clonage,
   - éventuellement un promoteur Ea fonctionnel dans les cellules eucaryotes, notamment de mammifères, situé immédiatement à côté (en 3' ou en 5') dudit promoteur, de telle sorte qu'un gène placé sous le contrôle dudit promoteur Ea soit également fonctionnel dans les cellules I hôtes de clonage par l'effet du promoteur P,
   - un polylinker contenant des sites rares de restriction,
   - éventuellement un autre polylinker permettant l'introduction de fragments d'ADN dans ledit vecteur,
- un vecteur B, basé sur un squelette de vecteur usuel de clonage dans des cellules II hôtes de clonage, présentant de 5' en 3' :
   - un polylinker contenant des sites rares de restriction,
   - éventuellement un promoteur Eb fonctionnel dans les cellules eucaryotes, notamment de mammifères,
   - un gène III conférant une résistance à un antibiotique III dans les cellules I hôtes de clonage, sous le contrôle éventuel du promoteur Eb,
   - éventuellement une séquence de poléadénylation,
   - un autre polylinker permettant l'introduction de fragments d'ADN dans ledit vecteur B,
   - un autre polylinker contenant des sites rares de restriction.

Les vecteurs A et B sont dérivés de vecteurs usuels utilisés pour le clonage de fragments d'ADN. L'homme du métier comprend le terme « dérivé », qui signifie notamment que le vecteur final possède globalement le même squelette (notamment les origines de réplication, et éléments stabilisateurs...) que le vecteur de départ dont il est dérivé. Il est important de noter que ceci signifie également que, de préférence, les éléments intergéniques sont conservés. Les modifications apportées dans le vecteur de base sont donc restreintes et ne modifient pas l'hôte de réplication du vecteur, ni ses propriétés fondamentales (nombre de copies, taille de l'insert, stabilité...). On peut toutefois envisager de changer les gènes de sélection (résistance aux antibiotiques), pour autant que ceci ne modifie pas les autres propriétés du vecteur.

Dans les modes de réalisation préférés de l'invention, les vecteurs A et B sont dérivés des vecteurs pUC 19, pBR322, pBluescript, ou les vecteurs pRs de levures.

Dans un mode de réalisation préféré, le vecteur B présente ledit promoteur Eb fonctionnel dans les cellules eucaryotes si aucun promoteur Ea fonctionnel dans les cellules eucaryotes n'est présent sur le vecteur A.

Dans un autre mode de réalisation, ni le vecteur A, ni le vecteur B ne présentent de promoteurs fonctionnels dans les cellules eucaryotes.

Dans un mode de réalisation préféré, le kit selon l'invention contient également les instructions permettant la mise en oeuvre d'un procédé selon l'invention.

### DESCRIPTION DES FIGURES

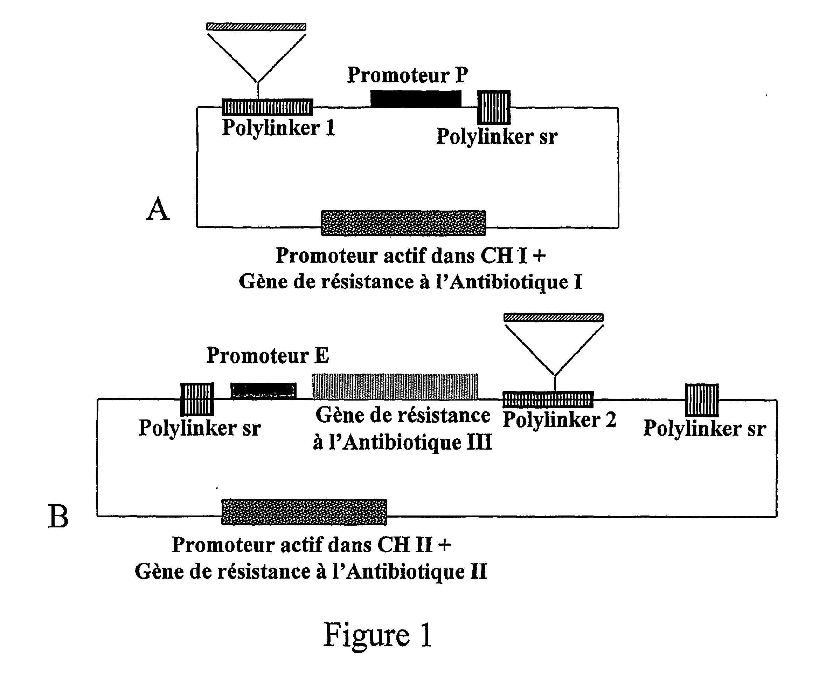

Figure 1 : Exemple de vecteurs A et B utilisables dans le procédé selon l'invention, selon l'exemple 1, version C. Le vecteur A induit la résistance à l'antibiotique I, mais pas à l'antibiotique III. Le vecteur B induit une résistance à l'antibiotique II, mais pas de résistance à l'antibiotique III. Polylinker sr : sites de restrictions) ; CH : cellules hôtes de clonage. Polylinkers 1 et 2 : permettent de cloner de l'ADN exogène, préférentiellement génomique.

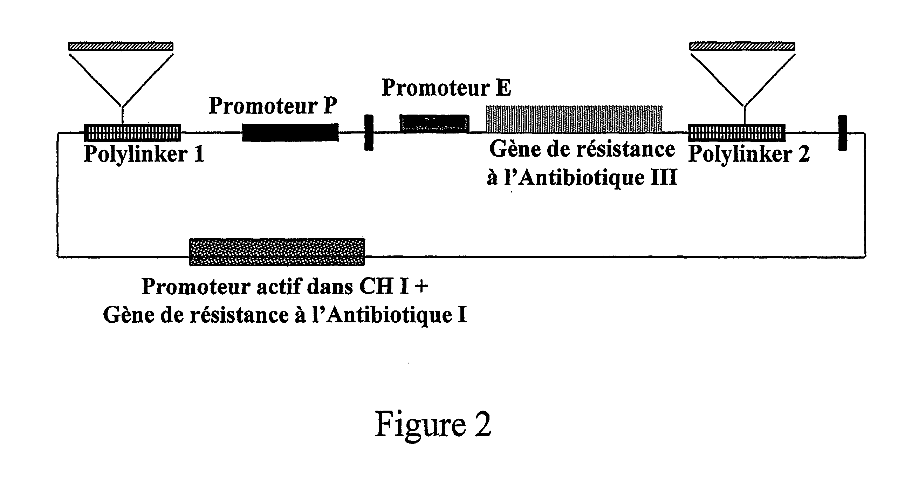

Figure 2: Vecteur final obtenu après digestion de A et B avec une enzyme de restriction située dans le polylinker SR, ligation de l'insert issu de B dans A, et sélection avec les antibiotiques I et III.

Les exemples d'application qui suivent ne servent qu'à illustrer certains aspects de l'invention, sans la restreindre en aucun cas.

### EXEMPLES

### Exemple 1 : Construction des plasmides selon l'invention

### Version A

Plasmide 1: Dans un squelette plasmidique de type pUC19 (résistant à l'ampicilline), on introduit de 5' en 3' :
- un polylinker permettant l'introduction de fragment d'ADN génomique
- un promoteur procaryote
- un promoteur eucaryote, couplé au promoteur procaryote
- un polylinker contenant des sites rares de restriction permettant l'insertion de l'insert provenant du plasmide 2.

Plasmide 2 : Dans un squelette plasmidique de type pUC19 (résistant à l'ampicilline), on introduit de 5' en 3' :
- un polylinker contenant des sites rares de restriction permettant l'excision de l'insert
- un ADNc codant pour la néomycine transférase ou tout autre gène de résistance actif en procaryotes et eucaryotes
- une séquence de polyadénylation
- un polylinker permettant l'introduction de fragment d'ADN génomique
- un polylinker contenant des sites rares de restriction permettant l'excision de l'insert.

### Version B

Plasmide 1 : Dans un squelette plasmidique de type pUC19 (résistant à l'ampicilline), on introduit de 5' en 3' :
- un polylinker permettant l'introduction de fragment d'ADN génomique
- un promoteur eucaryote
- un promoteur procaryote, couplé au promoteur eucaryote
- un polylinker contenant des sites rares de restriction permettant l'insertion de l'insert provenant du plasmide 2.

Plasmide 2 : Dans un squelette plasmidique de type pUC 19 (résistant à l'ampicilline), on introduit de 5' en 3' :
- un polylinker contenant des sites rares de restriction permettant l'excision de l'insert
- un ADNc codant pour la néomycine transférase ou tout autre gène de résistance actif en procaryotes et eucaryotes
- une séquence de polyadénylation
- un polylinker permettant l'introduction de fragment d'ADN génomique
- un polylinker contenant des sites rares de restriction permettant l'excision de l'insert..

### Version C

Plasmide 1 : Dans un squelette plasmidique de type pUC19 (résistant à l'ampicilline), on introduit de 5' en 3' :
- un polylinker permettant l'introduction de fragment d'ADN génomique
- un promoteur procaryote
- un polylinker contenant des sites rares de restriction permettant l'insertion de l'insert provenant du plasmide 2.

Plasmide 2 : Dans un squelette plasmidique de type pUC 19 (résistant à l'ampicilline), on introduit de 5' en 3' :
- un polylinker contenant des sites rares de restriction permettant l'excision de l'insert
- un promoteur eucaryote
- un ADNc codant pour la néomycine transférase ou tout autre gène de résistance actif en procaryotes et eucaryotes
- une séquence de polyadénylation
- un polylinker permettant l'introduction de fragment d'ADN génomique.

Il est à noter que l'on peut introduire, au lieu du gène codant pour la néomycine transférase, un gène codant pour un autre antibiotique (par exemple l'hygromycine), ou un gène marqueur tel le gène lacZ ou le gène de la GFP, dans une variante du procédé selon l'invention.

## Revendications

1. Procédé de clonage d'un fragment d'ADN dans un vecteur A comprenant un gène de résistance à un antibiotique I fonctionnel dans les cellules I hôtes de clonage, et un promoteur P fonctionnel dans les cellules I hôtes de clonage, comprenant les étapes consistant à :
a) intégrer de l'ADN dans le site polylinker d'un vecteur B utilisable dans des cellules II hôtes de clonage, ledit site polylinker étant localisé dans une cassette située entre deux sites de restrictions identiques ou différents, ladite cassette comprenant un gène III conférant une résistance à un antibiotique III dans les cellules I hôtes de clonage, ledit gène III n'étant pas sous le contrôle d'un promoteur lui permettant d'être actif dans les cellules I hôtes de clonage, ledit vecteur B présentant un gène II, actif dans les cellules II hôtes de clonage, de résistance à un antibiotique II,
b) exciser ladite cassette du vecteur B par coupure avec les enzymes de restriction correspondant auxdits sites de restriction,
c) effectuer une ligation de ladite cassette excisé dans ledit vecteur A linéarisé de telle sorte que ladite cassette est insérée sous le contrôle dudit promoteur I fonctionnel dans les cellules I hôtes de clonage,
d) sélectionner les événements de ligation chez les cellules I hôtes de clonage résistantes à la fois à l'antibiotique I et à l'antibiotique III,
*les cellules hôtes I et II étant des cellules non humaines.*

2. Procédé selon la revendication 1, **caractérisé en ce que** les cellules I hôtes de clonage et les cellules II hôtes de clonage sont des cellules procaryotes.

3. Procédé selon la revendication 1 ou 2, **caractérisé en ce que** ledit gène III confère également une résistance à un antibiotique dans des cellules eucaryotes notamment des cellules de mammifères.

4. Procédé selon l'une des revendications 1 à 3, **caractérisé en ce que** lesdits gènes I et II sont identiques.

5. Procédé selon l'une des revendications 1 à 4, **caractérisé en ce que** lesdits sites de restriction sont rares.

6. Procédé selon l'une des revendications 1 à 5, **caractérisé en ce que** ledit vecteur A possède également un site polylinker permettant ou ayant permis l'insertion d'un fragment d'ADN.

7. Procédé selon l'une des revendications 2 à 6, **caractérisé en ce que** ledit promoteur P est choisi parmi le promoteur pour le gène de résistance à la kanamycine du transposon Tn5, le promoteur du gène bla (résistance à l'ampicilline), le promoteur de l'opéron tryptophane Trp, le promoteur de l'opéron lactose, ou tout autre promoteur accessible dans la base de données PromEC.

8. Procédé selon l'une des revendications 1 à 7, **caractérisé en ce que**
- ledit gène III dans ladite cassette dans le vecteur B est sous la dépendance d'un promoteur Eb actif dans les cellules eucaryotes, notamment mammifères, ou
- ledit vecteur A présente un promoteur Ea actif dans les cellules eucaryotes, notamment mammifères, situé de telle sorte que ledit gène III est sous le contrôle dudit promoteur Ea après insertion dans le vecteur A.

9. Procédé selon la revendication 8, **caractérisé en ce que** ledit promoteur E est choisi parmi les promoteurs de la bêta-actine de poulet, du PGK, de la thymidine kinase du virus d'herpes simplex, du SV40, ou bien le « immédiate early enhancer » du cytomégalovirus humain.

10. Procédé selon l'une des revendications 1 à 8, **caractérisé en ce que** les fragments d'ADN introduits dans ledit vecteur B et de façon optionnelle dans ledit vecteur A sont des fragments d'ADN génomique issus d'un même hôte.

11. Procédé selon la revendication 10, **caractérisé en ce que** lesdits fragments sont les fragments 3' et 5' d'un même locus.

12. Kit pour le clonage de fragments d'ADN dans un vecteur A, comprenant :
- un vecteur A présentant de 5' en 3', sur un squelette de vecteur usuel de clonage dans des cellules I hôtes de clonage, ledit vecteur comprenant un gène de résistance à un antibiotique I fonctionnel dans lesdites cellules I :
- éventuellement un polylinker permettant l'introduction de fragments d'ADN dans ledit vecteur,
- un promoteur P fonctionnel dans les cellules I hôtes de clonage,
- éventuellement un promoteur Ea fonctionnel dans les cellules eucaryotes, notamment de mammifères, situé immédiatement à côté (en 3' ou en 5') dudit promoteur,
- un polylinker contenant des sites rares de restriction,
- éventuellement un autre polylinker permettant l'introduction de fragments d'ADN dans ledit vecteur,
- un vecteur B présentant de 5' en 3', sur un squelette de vecteur usuel de clonage dans des cellules II hôtes de clonage :
- un polylinker contenant des sites rares de restriction,
- éventuellement un promoteur Eb fonctionnel dans les cellules eucaryotes, notamment de mammifères,
- un gène III conférant une résistance à un antibiotique III dans les cellules I hôtes de clonage, sous le contrôle éventuel du promoteur Eb, *ledit gène III n'étant pas sous le contrôle d'un promoteur lui permettant d'être actif dans les cellules I hôtes de clonage,*
- éventuellement une séquence de poléadénylation,
- un autre polylinker permettant l'introduction de fragments d'ADN dans ledit vecteur B,
- un autre polylinker contenant des sites rares de restriction.

13. Kit selon la revendication 12, **caractérisé en ce que** le vecteur B présente ledit promoteur Eb fonctionnel dans les cellules eucaryotes si aucun promoteur Ea fonctionnel dans les cellules eucaryotes n'est présent sur le vecteur A.

14. Kit selon l'une des revendications 12 ou 13, pour la mise en oeuvre d'un procédé selon l'une des revendications 1 à 11.

## Claims

1. A method for cloning a DNA fragment in a vector A, comprising a functional antibiotic I resistance gene in the cloning host cells I, and a promoter P functional in the host cloning cells I, comprising the steps consisting of:
a) integrating the DNA into the polylinker site of a vector B usable in cloning host cells II, said polylinker site being located in a cassette situated between two identical or different restriction sites, said cassette comprising a gene III providing resistance to an antibiotic III in the cloning host cells I, said gene III not being under the control of a promoter, thus enabling it to be active in the cloning host cells I, said vector B having a gene II active in the cloning host cells II, providing resistance to an antibiotic II,
b) excising said cassette from the vector B by cutting with restriction enzymes corresponding to said restriction sites,
c) ligating said excised cassette in said linearized vector A, whereby said cassette is inserted under the control of said promoter I functional in the cloning host cells I,
d) selecting the ligation events in the cloning host cells I resistant to both antibiotic I and antibiotic III, wherein host cells I and II are non-human cells.

2. A method of claim 1, **characterised in that** cloning host cells I and cloning host cells II are prokaryote cells.

3. A method as claimed in claim 1 or 2, **characterised in that** said gene III also provides resistance to an antibiotic in eukaryote cells, in particular mammalian cells.

4. A method as claimed in one of claims 1 to 3, **characterised in that** said genes I and II are identical.

5. A method as claimed in one of claims 1 to 4, **characterised in that** said restriction sites are rare.

6. A method as claimed in one of claims 1 to 5, **characterised in that** said vector A also possesses a polylinker site enabling or having enabled the insertion of a DNA fragment.

7. A method as claimed in one of claims 2 to 6, **characterised in that** said promoter P is chosen from among the promoters of the kanamycin resistance gene from transposon Tn5, the promoter of the bla gene (ampicillin resistance), the promoter of the tryptophan operon Trp, the promoter of the lactose operon, or any other promoter accessible in the PromEC database.

8. A method as claimed in one of claims 1 to 7, **characterised in that**:
- said gene III in said cassette in vector B is under the control of a promoter Eb active in eukaryote cells, in particular mammalian cells, or
- said vector A has a promoter Ea active in eukaryote cells, in particular mammalian cells, which is situated such that said gene III is under the control of said promoter Ea, after insertion into vector A.

9. A method of claim 8, **characterised in that** said promoter E is chosen from among the promoters of the chicken beta-actin gene, PGK, herpes simplex virus thymidine kinase, SV40, or else the "immediate early enhancer" of the human cytomegalovirus.

10. A method as claimed in one of claims 1 to 8, **characterised in that** the DNA fragments introduced into said vector B, and optionally into said vector A, are genomic DNA fragments originating from a single host.

11. A method of claim 10, **characterised in that** said fragments are 3' and 5' fragments from a single locus.

12. Kit for cloning DNA fragments into a vector A, comprising:
-- a vector A having from 5' to 3', on a ordinary vector skeleton used in cloning host cells I, said vector comprising an antibiotic resistance gene I functional in said cells I:
- possibly a polylinker enabling the introduction of DNA fragments into said vector,
- a promoter P functional in the cloning host cells I,
- possibly a promoter Ea functional in eukaryote cells, in particular mammalian cells, situated immediately adjacent to said promoter(at 3' or at 5'),
- a polylinker containing rare restriction sites,
- possibly another polylinker enabling the introduction of DNA fragments into said vector,
-- a vector B having from 5' to 3' , on an ordinary vector skeleton used in cloning host cells II:
- a polylinker containing rare restriction sites,
- possibly a Eb promoter functional in eukaryote cells, in particular mammalian cells,
- a gene III providing resistance to an antibiotic III in cloning host cells I, under the possible control of the promoter Eb, said gene III not being under the control of a promoter enabling it to be active in the cloning host cells I,
- possibly a polyadenylation sequence,
- another polylinker enabling the insertion of DNA fragments into said vector B,
- another polylinker containing rare restriction sites.

13. A kit of claim 12, **characterised in that** the vector B presents said promoter Eb functional in eukaryote cells, if no promoter Ea functional in eukaryote cells is present on vector A.

14. A kit as claimed in one of claims 12 or 13, for implementing a method as claimed in one of claims 1 to 11.

## Patentansprüche

1. Verfahren zur Klonierung eines DNA-Fragments in einen Vektor A, der ein eine Resistenz gegen ein Antibiotikum I verleihendes Gen, welches in den Klonierungswirtszellen I funktionsfähig ist, und einen Promotor P umfasst, welcher in den Klonierungswirtszellen I funktionsfähig ist, umfassend die Schritte, die darin bestehen:
a) die DNA in die Polylinkerstelle eines Vektors B, welcher in Klonierungswirtszellen II einsetzbar ist, zu integrieren, wobei die Polylinkerstelle in einer Kassette lokalisiert ist, welche sich zwischen zwei identischen oder unterschiedlichen Restriktionsstellen befindet, wobei die Kassette ein Gen III umfasst, welches eine Resistenz gegen ein Antibiotikum III in den Klonierungswirtszellen I verleiht, wobei das Gen III nicht unter der Kontrolle eines Promotors steht, welcher es diesem erlaubt, in den Klonierungswirtszellen I aktiv zu sein, wobei der Vektor B ein eine Resistenz gegen ein Antibiotikum II verleihendes Gen II aufweist, das in den Klonierungswirtszellen II aktiv ist,
b) die Kassette aus dem Vektor B durch Schneiden mit den Restriktionsenzymen, welche den Restriktionsstellen entsprechen, herauszuschneiden,
c) eine Ligation der herausgeschnittenen Kassette in den linearisierten Vektor A derart auszuführen, dass die Kassette unter der Kontrolle des in den Klonierungswirtszellen 1 funktionsfähigen Promotors I inseriert wird,
d) die Ligationsereignisse in den Klonierungswirtszellen I, die zugleich gegen das Antibiotikum I und das Antibiotikum III resistent sind, zu selektieren,
wobei die Wirtszellen I und II nicht-humane Zellen sind.

2. Verfahren nach Anspruch 1, **dadurch gekennzeichnet, dass** die Klonierungswirtszellen I und die Klonierungswirtszellen II prokaryotische Zellen sind.

3. Verfahren nach Anspruch 1 oder 2, **dadurch gekennzeichnet, dass** das Gen III gleichfalls eine Resistenz gegen ein Antibiotikum in eukaryotischen Zellen, insbesondere Säugerzellen, verleiht.

4. Verfahren nach einem der Ansprüche 1 bis 3, **dadurch gekennzeichnet, dass** die Gene I und II identisch sind.

5. Verfahren nach einem der Ansprüche 1 bis 4, **dadurch gekennzeichnet, dass** die Restriktionsstellen seltene sind.

6. Verfahren nach einem der Ansprüche 1 bis 5, **dadurch gekennzeichnet, dass** der Vektor A gleichfalls eine Polylinkerstelle aufweist, welche die Insertion eines DNA-Fragments zulässt oder zugelassen hat.

7. Verfahren nach einem der Ansprüche 2 bis 6, **dadurch gekennzeichnet, dass** der Promotor P unter dem Promotor für das Kanamycinresistenzgen des Transposons Tn5, dem Promotor des bla-Gens (Ampicillinresistenz), dem Promotor des Tryptophan-Operons Trp, dem Promotor des Lactose-Operons oder einem jeglichen anderen Promotor, der in der Datenbank PromEC zugänglich ist, ausgewählt ist.

8. Verfahren nach einem der Ansprüche 1 bis 7, **dadurch gekennzeichnet, dass**
- das Gen III in der Kassette in dem Vektor B unter der Abhängigkeit eines Promotors Eb steht, welcher in den eukaryotischen Zellen, insbesondere Säugerzellen aktiv ist, oder
- der Vektor A einen in den eukaryotischen Zellen, insbesondere Säugerzellen aktiven Promotor Ea aufweist, welcher derart gelegen ist, dass das Gen III unter der Kontrolle dieses Promotors Ea nach Insertion in den Vektor A steht.

9. Verfahren nach Anspruch 8, **dadurch gekennzeichnet, dass** der Promotor E unter den Promotoren des Beta-Aktins vom Huhn, von PGK, der Thymidinkinase des Herpes simplex-Virus, von SV40 oder auch dem "immediate early enhancer" des humanen Zytomegalievirus ausgewählt ist.

10. Verfahren nach einem der Ansprüche 1 bis 8, **dadurch gekennzeichnet, dass** die in den Vektor B und optional in den Vektor A eingeführten DNA-Fragmente genomische DNA-Fragmente sind, die von ein und demselben Wirt stammen.

11. Verfahren nach Anspruch 10, **dadurch gekennzeichnet, dass** die Fragmente die 3'- und 5'-Fragmente von ein und demselben Genort sind.

12. Kit für die Klonierung von DNA-Fragmenten in einen Vektor A, umfassend:
- einen Vektor A, welcher in einem üblichen Vektorgerüst für die Klonierung in Klonierungswirtszellen I von 5' nach 3' aufweist:
- gegebenenfalls einen Polylinker, welcher die Einführung von DNA-Fragmenten in den Vektor erlaubt,
- einen Promotor P, welcher in den Klonierungswirtszellen I funktionsfähig ist,
- gegebenenfalls einen Promotor Ea, welcher in den eukaryotischen Zellen, insbesondere von Säugern, funktionsfähig ist und sich unmittelbar neben (3' oder 5') dem Promotor befindet,
- einen Polylinker, welcher seltene Restriktionsstellen enthält,
- gegebenenfalls einen anderen Polylinker, welcher die Einführung von DNA-Fragmenten in den Vektor erlaubt,
wobei der Vektor ein eine Resistenz gegen ein Antibiotikum I verleihendes Gen umfasst, welches in den Zellen I funktionsfähig ist,
- einen Vektor B, welcher in einem üblichen Vektorgerüst für die Klonierung in Klonierungswirtszellen II von 5' nach 3' aufweist:
- einen Polylinker, welcher seltene Restriktionsstellen enthält,
- egebenenfalls einen Promotor Eb, der in den eukaryotischen Zellen, insbesondere von Säugern, funktionsfähig ist,
- in Gen III, welches eine Resistenz gegen ein Antibiotikum III in den Klonierungswirtszellen I verleiht, unter der etwaigen Kontrolle des Promotors Eb, wobei das Gen III nicht unter der Kontrolle eines Promotors steht, welcher es diesem erlaubt, in den Klonierungswirtszellen I aktiv zu sein,
- gegebenenfalls eine Polyadenylierungssequenz,
- inen weiteren Polylinker, welcher die Einführung von DNA-Fragmenten in den Vektor B erlaubt,
- einen weiteren Polylinker, welcher seltene Restriktionsstellen enthält.

13. Kit nach Anspruch 12, **dadurch gekennzeichnet, dass** der Vektor B den in den eukaryotischen Zellen funktionsfähigen Promotor Eb aufweist, wenn kein in den eukaryotischen Zellen funktionsfähiger Promotor Ea in dem Vektor A vorhanden ist.

14. Kit nach einem der Ansprüche 12 oder 13 für das Ausführen eines Verfahrens nach einem der Ansprüche 1 bis 11.
